(19) **Europäisches Patentamt European Patent Office Office européen des brevets**

(11) **EP 1 652 517 B1**

(12) **EUROPEAN PATENT SPECIFICATION**

(45) Date of publication and mention of the grant of the patent:
**22.02.2012 Bulletin 2012/08**

(51) Int Cl.:
*A61K 9/51* (2006.01)  *C08B 37/08* (2006.01)

(21) Application number: **04736983.0**

(22) Date of filing: **17.06.2004**

(86) International application number:
**PCT/ES2004/000284**

(87) International publication number:
**WO 2004/112758 (29.12.2004 Gazette 2004/53)**

(54) **HYALURONIC ACID NANOPARTICLES**

HYALURONSÄURE-NANOPARTIKEL

NANOPARTICULES D'ACIDE HYALURONIQUE

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **20.06.2003 ES 200301456**

(43) Date of publication of application:
**03.05.2006 Bulletin 2006/18**

(73) Proprietor: **Advanced in Vitro Cell Technologies, S.L.**
**08028 Barcelona (ES)**

(72) Inventors:
• **ALONSO FERNANDEZ, Maria, José**
**E-15782 Santiago de Compostela (ES)**
• **DE LA FUENTE FREIRE, Maria**
**E-15782 Santiago de Compostela (ES)**
• **SEIJO REY, Maria, Begona**
**E-15782 Santiago de Compostela (ES)**

(74) Representative: **ABG Patentes, S.L.**
**Avenida de Burgos 16D**
**Edificio Euromor**
**28036 Madrid (ES)**

(56) References cited:
**WO-A1-2004/009060    ES-A1- 2 098 188**
**ES-A1- 2 114 502    FR-A1- 2 642 329**

• **LIM S.T. ET AL.: 'Preparation and evaluation of the in vitro drug release properties and mucoadhesion of novel microspheres of hyaluronic acid and chitosan' J. CONTROL. REL. vol. 66, no. 2-3, May 2000, pages 281 - 292, XP002291345**

Remarks:
The file contains technical information submitted after the application was filed and not included in this specification

**Description**

## FIELD OF THE INVENTION

The invention relates to the development of a nanoparticulate system for the administration of active macromolecules, both hydrophilic and hydrophobic, a composition which comprises same and a method for their preparation. These nanoparticles comprise hyaluronic acid in salt form, preferably, the sodium salt of said polymer, and a positively-charged polymer, preferably chitosan. A polyanionic salt is incorporated in said formulation, preferably selected from the phosphates group. These nanoparticles can be used for the administration of active ingredients to the organism by different routes. The active ingredients can be molecules with therapeutic properties, vaccinations or cosmetic ingredients.

## BACKGROUND OF THE INVENTION

The administration of active ingredients presents numerous difficulties, both depending on the administration route used and the physicochemical and morphological characteristics of the molecules. It is known that the main drawbacks arise when administering unstable active molecules, which are hydrophilic and large-sized. Furthermore, access of hydrophilic macromolecules to the interior of the organism is limited by the low permeability of the biological barriers. Likewise, they are susceptible of being degraded due to the different defence mechanisms both human and animal organisms have. These difficulties have to be resolved to achieve access of the active molecule to the therapeutic target and, thus, effective treatment.

It has been demonstrated that the incorporation of macromolecules in nanometric-sized systems makes it easier for them to penetrate the epithelial barriers and protects them from being degraded. Thus, the design of nanoparticulate systems capable of interacting with said barriers is presented as a promising strategy in order to achieve the penetration of active ingredients through mucous membranes.

It is also known that thc capacity of these systems Lo cross external barriers and access the interior of the organism, both depends on their size and on their composition. Small-sized particles will increase the degree of transport with respect to those of larger size; nanoparticles, with diameter less than 1 $\mu$m, respond to this criteria. If they are prepared from polymers of natural, biocompatible and biodegradable origin, The possibilities increase of them being naturally transported through the organism's mucous membranes, by known transport mechanisms and without altering the epithelials' physiology. Another characteristic of nanoparticulate systems is that they permit the controlled release of the active molecules they incorporate and their orientation towards the target tissue.

Hyaluronic acid is a polymer of natural origin. More specifically, it is a glycosaminoglycan present in the extracellular matrix of connective tissues, such as subcutaneous tissue and cartilage, as well as in the vitreous body of the ocular globe and in the synovial fluid of articular cavities. It is a polymer for which there exist receptors, CD44 and RHAMM being predominant, which are located in the cell surface in practically all the organism's cells, with the exception of red blood cells. The interaction of hyaluronic acid with these receptors allows certain physiological processes such as mobility and cell proliferation to be regulated. Due to these properties, hyaluronic acid has therapeutic use, as it plays an important role in processes such as morphogenesis and embryo development, cancer and inflammation. Furthermore, due to said properties, hyaluronic acid is used to promote epithelial healing. Proof of this biological activity are the numerous works that include hyaluronic acid as active biomolecule, for example, those described by Sand et al., Acta Ophthalmol. 67, 1989, 181-183, where hyaluronic acid is applied in the treatment of keratoconjuntivitis sicca and Nishida et al., Exp. Eye Res 53, 1991, 753-758, where it is applied as a wound healing agent in the cornea.

Hyaluronic acid and its derivatives, presented in different forms, have been object of numerous patents. In some of these documents, hyaluronic acid is presented as an active molecule and in others as biomaterial excipient used in the development of drug-release systems. Its interest in this line is due to it being a biodegradable, biocompatible polymer, which is not immunogenic and has mucoadhesive properties.

Among the **patents wherein hyaluronic acid is quoted as an example of active molecule, we should highlight the following:**

Document WO9606622 claims the use of hyaluronic acid and derivatives, alone or in combination with another therapeutic agent, to modulate Lhe cellular activity of those tissues and cells which express receptors for hyaluronic acid on their surface, and thus treat or prevent inflammatory processes, fibrosis or oncogenesis.

Patent US6383478 protects a release system consisting of microparticles, nanoparticles or films which incorporate hyaluronic acid as possible active molecule to promote angiogenesis. The polymeric film or particulate vehicle are formed by at least two anionic polymers (among which hyaluronic acid is not included), a cationic polymer (among which neither chitosan, gelatine nor collagen are included) and a low-molecular weight cation.

Document WO0101964 relates to the formation of an ionic complex, among hydrophilic polymers of opposite charges, which will later be precipitated, giving rise Lo particle formation, in a size range between 5nm lmm. The cationic polymer may be a polymer with a positive charge, such as, for example, chitosan. Dextran sulphate and others are mentioned as anionic polymers. Precipitation takes place when the complex is desolvated, by the addition of desolvating agents, in this case, zinc sulphate. These particulated complexes incorporate a biomolecule which is previously chelated with one of the hydrophilic polymers that form part of the vehicle. Hyaluronic acid may be one of the biomolecules incorporated, as the description includes the use of polysaccharides. Therefore, it is a system wherein the active molecule (hyaluronic acid) is chelated with a cationic polymer (e.g. chitosan) and this complex is made to interact with another anionic polymer(dextran sulphate) and the unit is precipitated by adding zinc sulphate.

Document WO9/04747 discloses the preparation of nanoparticles from principally hydrophobic polymers, said nanoparticles being coated by an adhesive agent. As an example of an active polymer it cites a polysaccharide, which may be hyaluronic acid, although it is not explicitly mentioned. Although said patent indicates chitosan as possible material to form the nanoparticles, all The examples relate to the use of hydrophobic polymers, said organic solvents being necessary to form the nanoparticles.

The group of patents wherein **hyaluronic acid is used as an excipient for the development of active ingredient-release systems,** is also very extensive. Said systems may be presented in the form of simple complexes, hydrogels, microspheres and nanoparticles.

Among the numerous systems which incorporate hyaluronic acid or derivatives thereof in their composition, we should highlight the following documents:

Document EP0544259 relates to the preparation of a hyaluronic acid **complex** with a high molecular weight material with amino groups which may be chitosan. This complex is present in different forms, adopting that of the recipient wherein it is obtained.

Document WO018274 claims a composition which is a simple particulate **complex** formed from a positively-charged aminopolysaccharide, which may be chitosan, and a negatively-charged polysaccharide, mentioning hyaluronic acid. This particulate complex is formed according to a method of uncontrolled precipitation. In other words, no crosslinking agents are used which allow the formation of particles to be controlled, for which reason the resulting particles are normally irregular and highly dispersed.

Furthermore, there is a series of patents which protect the production of **hydrogels** in accordance with different methods and compositions. Among these we should highlight:

US4582865 protects the preparation of hyaluronic acid hydrogels or derivatives, alone or in combination with other hydrophilic polymers, such as cellulose, collagen, xanthan, carboxymethylcellulose, etc. obtained when making them react with a divinylsulphone.

WO0128602 discloses the preparation of an injectable formula, in the form of gel or paste, for the release of osteogenic proteins which comprise benzyl ester derivatives of hyaluronic acid, an osteogenic protein and calcium triphosphate as mineral component.

Document WO9009401 relates to hyaluronic acid hydrogels or derivatives, obtained by polymer crosslinking after making it react with a phosphoric acid derivative, where phosphate ester bridges are established. These hydrogels are useful for application as active ingredient-deposit implants, in the form of films, tubes, etc.

Document WO0230990 discloses the production of a crosslinked amide derivative of hyaluronic acid, based on a reaction thereof with a cationic polymer with two or more amine groups (including chitosan). A carboxylic group activating agent is necessary to perform this chemical reaction, using a carbodiimide. This amide derivative of hyaluronic acid can be present in the form of gels, membranes, beads...

Likewise, a series of documents exist which make reference to the production of **particles (microparticles or nanoparticles)** which include hyaluronic acid in their composition. We should make a distinction between microspheres or microparticles, whose particle size is between 1-100 μm, and nanospheres or nanoparticles, whose size is less than one micron. Although patents exist that claim very wide particle size ranges (from nano to micro), that many of the technologies applicable to producing microparticles do not allow nanoparticles to be formed.

Thus, patent WO 89/03207 and the article by Benedetti et al., Journal of Controlled Release 13, 33-41 (1990) show the production of hyaluronic acid microspheres according to the solvent evaporation method. More recently, document US6066340 relates to the possibility of obtaining said microspheres making use of solvent extraction techniques. Nevertheless, said documents do not mention the production of nanoparticles as is it not possible to achieve the formation of nanoparticles according to the techniques referred to therein.

Furthermore, the combination of hyaluronic acid and chitosan in a microparticulate system have been proposed with the aim of combining the mucoadhesive effect of hyaluronic acid with the chitosan absorption promoting effect. The value of this microparticulate combination is reflected in the works by Lim et al., J. Controll. Rel. 66, 2000, 281-292 *and* Lim et al., Tnt. J. Pharm. 23, 2002, 73-82. As with the previous document, these microparticles have been prepared by the solvent emulsion-evaporation Lcchnique.

Document US2001053359 proposes the combination, for nasal administration, of an antiviral and a bioadhcsive material, being presented in the form of a solution or microspheres comprised of different materials, among others, gelatine, chitosan or hyaluronic acid, but not mixtures thereof. The microparticles are obtained by classic techniques such as atomising and solvent emulsion/evaporation. Once obtained, the microparticles are hardened by conventional chemical crosslinking methods (dialdehydes and dicetones).

DocumenL US2002197328 also relates to microparticles, prepared from hyaluronic acid by atomising. The difference with respect to the previous is that the high-molecular weight hyaluronic acid microparticles (over 1,000,000 Daltons) are protected. Although the claims indicate the preparation of particles of less than 1 micron, the atomising process whereby said particles are obtained does not allow nanoparticles to be obtained.

More recently, US20030026844 has been geared towards protecting porous particles, of a size between 10nm -500$\mu$m, which have functional ionic groups on their surface. These particles are formed from one or more biopolymers (which include determined polysaccharides such as hyaluronic acid and chitosan). According to this document, the ionic groups are achieved thanks to the essential incorporation of ionisable surfactant agents. Different methods are disclosed for the formation of these particles, such as solvent extraction or evaporation, atomising, coacervation and use of supercritical fluids. Despite the claims indicating the preparation of particles with size smaller than 1 micron, the methods disclosed in said document do not allow nanoparticles to be obtained.

Document WO-A-99/47130 relates to nanoparticles which have a polyelectrolytic complex, from polycation (which may be chitosan) and a polyanion, as well as at least one bioactive ingredient, the nanoparticles being obtainable by additionally treating the polyelectrolytic complex during and after their formation with at least one crosslinking agent (glioxal, TST0 or EDAP). Polysilane sulphate is indicated as polyanion.

Document US6132750 relates to the preparation of small-sized particles (micro and nanoparticles) which contain at least one protein (collagen, gelatine) and to a polysaccharide (chitosan or glycosaminoglycans, among others) on their surface. They are formed by interfacial crosslinking with a polyfunctional acylating agent which forms amide or ester bonds, and optionally anhydrous bonds. It is aimed that free groups remain on its surface capable of reacting with metal ions.

Document WO9918934 relates to nanoparticles which consist of a nucleus formed from a positively- or negatively-charged polymer and a coating form from the combination of both. Ultrasounds need to be applied during the production method thereof. The particles are stabilised by the reaction thereof with a crosslinking agent (a dextran polyaldehyde, a photocrosslinking polymer or a glutamil transferase).

## SUMMARY OF THE INVENTION

The present invention relates to nanoparticles which comprise hyaluronic acid in salt form, preferably the sodium salt of said polymer, and a positively-charged polymer of natural origin, preferably chitosan, so that it electrostatically interacts with the deprotonated form of hyaluronic acid. A polyanionic salt is incorporated in the formulation, capable of ionically crosslinking the cationic molecule, causing its gelling, preferably selected from the phosphates group.

A combination of hyaluronic acid and chitosan, in nanoparticulate form, leads to a system being obtained with high potential in the therapeutic field. Furthermore, the possibility of obtaining ionic complexes from both polymers is known, as they have opposite charges. The difference is also known between complexes and nanoparticles, as the advantage of nanoparticles with respect to complexes is greater control with respect to their composition and size, as well as greater stability. In order to provide stability to the systems, they have been crosslinked by adding substances which form chemical bonds between the compounds.

Due to the aforementioned, the present invention relates to the combination of two polymers, hyaluronic acid and chitosan, being able to substitute chitosan for other positively-charged polymers of natural origin, such as collagen and gelatine, to obtain a nanoparticulate system. Likewise, a method has been found for the preparation of nanoparticles which gives rise to the formation of same in a controlled manner and which dispenses with the use of organic solvents as well as extreme conditions. Therefore, it thus preserves the integrity of the macromolecules incorporated in the system, which is susceptible to be degraded. To achieve the formation of nanoparticles in a desired size range, it resorts to the addition of a polyanionic salt which will lead to the gelling of the positively-charged polymer, simultaneous with the ionic interaction with hyaluronic acid. It is, therefore, an ionic gelling/interaction method which occurs in a controlled manner and will provide stability to the system, without the need to create covalent bonds between The components. These nanoparticles will have advantages with respect to other systems of greater size (microparticles, pellets, vedas, films, sponges...) with regard to their biological applications. Indeed, it is known that the interaction of a drug-release system with a biological surface is highly conditioned by its size. Thus, nanoparticles are capable of crossing epithelials and mucous membranes acting as drug transport systems, whilst microparticles do not have that capacity. The biodistribution of these systems is also highly conditioned by size. The knowledge generated in recent years in drug-release colloidal systems has allowed a clearly defined frontier to be set between the colloidal systems (less than one micron) and microparticulate systems.

## DESCRIPTION OF THE INVENTION

The present invention discloses the preparation of nanoparticles formed from a hyaluronic acid salt and another hydrophilic polymer capable of interacting with said glycosaminoglycan, said interaction being mediated by a polyanionic salt capable of crosslinking the system by establishing electrostatic interactions. The method to obtain the particles is a simple method which avoids the use of organic solvents as well as drastic conditions. Furthermore, neither is it necessary to perform any type of chemical reaction to obtain same, as the crosslinking process is ionic, as has been indicated.

According to a first aspect, the present invention relates to a method of obtaining hyaluronic acid nanoparticles with a diameter less than 1$\mu$m, which incorporate an active ingredient, irrespective of the hydrophobic or hydrophilic nature thereof. This method comprises the following steps:

    a) preparing an aqueous solution of a hyaluronic acid salt, preferably in a concentration of between 0.50 and 5 mg/mL;
    b) preparing an aqueous solution of a cationic polymer, preferably in a concentration of between 0.50 and 5 mg/mL;
    c) adding a polyanionic salt to the solution of the hyaluronic acid salt, preferably in a concentration of between 0.25 and 1.00 mg/mL;
    d) stir-mixing the solutions resulting from steps b) and c), spontaneously obtaining the nanoparticles.

The active ingredient or active ingredients are dissolved in one of solutions a), b) or c) or in the suspension of nanoparticles obtained in step d) to be adsorbed on the nanoparticles.

According to a second aspect, the present invention relates to nanoparticles obtained according to the preceding method, with determined characteristics with regard to its composition, properties and morphology, comprising hyaluronic acid, a positively-charged polymer, a polyanionic salt and a macromolecule.

According to an additional aspect, the invention relates to a pharmaceutical or cosmetic composition which comprises the previous nanoparticles, together with one or more pharmaceutically or cosmetically acceptable excipients, respectively.

According to a preferred embodiment, the hyaluronic acid salt is the sodium salt thereof. Preferably, the positively-charged polymer will be chitosan, also being possible to use collagen or gelatine.

Also preferably, the polyanionic salt will be selected from the phosphates group, taking the sodium triphosphate as model due to the high number of negative charges its structure has.

The particles are formed by mixing volumes of said solutions in different proportions. In this way, the nanoparticles will have a proportion relative to the different hyaluronic acid:positive polymer:anionic salt ingredients which may vary between 1:0.5:0.1 and 1:10:2 and, preferably, between 1:1:0.15 and 1:10:1.5.

The method of preparing the hyaluronic acid particles may include an additional lyophilisation stage, with the aim of preserving them during their storage so that they preserve their initial characteristics. In lyophilised form, the nanoparticles may be stored for long periods of time, and be easily regenerated, when necessary, simply by adding an optimum volume of water. Furthermore, the degree of crosslinking of the nanoparticles increases with this method, as an approximation takes place between the polymeric chains, which facilitates the increase in polymeric crossover, as well as boosting the effect of the polyanion as a crosslinking agent.

For particle lyophilisation, it is only necessary to add small quantities of sugars, as hyaluronic acid exerts a cryoprotective effect.

In accordance with this additional stage, the present invention also relates to hyaluronic acid nanoparticles and a positive polymer in lyophilized form and a pharmaceutical or cosmetic composition which includes them, as well as at least one pharmaceutically or cosmetically acceptable excipient.

The nanoparticles disclosed herein have suitable stability both in suspension and in lyophilized form, for which reason they can be stored for long periods of time. Furthermore, their stability has also been studied in certain biological fluids which guarantee that they will remain in nanoparticulate form after their administration to human or animal organisms.

Furthermore, the nanoparticles that comprise hyaluronic acid in their composition have demonstrated having excellent mucoadhesive properties due to their capacity of interaction with mucin (protein present in mucous),which converts them in systems of great use as pharmaceutical or cosmetic systems. They may be administered by different routes, and among them mucous membrane administration is highly important, as well as their administration by intra-articular injection.

The active ingredient to be incorporated in the nanoparticles comprising hyaluronic acid will have suitable pharmacotherapeutical properties for the therapeutic application for which the formulation is intended. The effect of the incorporated macromolecules on the human or animal organism will have the object of curing, minimising or preventing an illness, after being administered.

According to the present invention, the hyaluronic acid nanoparticles and a cationic polymer, such as chitosan, are suitable for incorporating macromolecules irrespective of the solubility characteristics thereof. The association capacity will depend on the macromolecule incorporated, but in general terms it will be high both for hydrophilic macromolecules and for those of marked hydrophobic character. The active ingredient can be a drug, a vitamin, a vaccination, etc. or a cosmetic agent.

The macromolecule designed to be incorporated in nanoparticles will previously be dissolved in one of the two aqueous solutions which are used in the production thereof. In the case of macromolecules of lypophilic character, a variant has been introduced in the production technique according to which the active ingredient is dissolved in a small volume of a mixture of water and a water-miscible organic solvent, preferably acetronitrile, preferably in an approximate proportion of 1:1, which will then be added to one of the aforementioned aqueous solutions, so that the concentration by weight of the organic solvent in the end solution is always less than 10%.

There is the possibility of the nanoparticles disclosed in the present invention incorporating more than one macroparticle, which may be dissolved in the same solution or in both separately, this depending on the macromolecules to be incorporated, avoiding any type of interaction, either chemical or physical, from existing.

The hyaluronic acid nanoparticles have a mean diameter of less than 1 μm, therefore responding to the definition of nanoparticles, colloidal System formed from polymers with a size less than 1 μm. The size thereof will vary in accordance with the quantity of hyaluronic acid that constitutes them, as well as in accordance with the quantity of polyanionic salt which is used in the system crosslinking, and the nature of the active ingredient they include.

The surface charge thereof can vary in accordance with the different proportions of the polymers comprising them. More specifically, the surface charge of the nanoparticles varies in magnitude in accordance with the quantity of hyaluronic acid which comprises them, and with the crosslinking polyanionic salt. Frequently, it is of interest that the surface charge takes on positive values, as the biological surfaces of the organism, and particularly the mucous membranes, are negatively charged. Therefore, the positive charge of the nanoparticles favours their interaction with same, and, consequently, it will favour the macromolecules associated to the nanoparticulate system acting on the target tissues.

The quantity of hyaluronic acid included in the formation of these nanoparticles is further expected to modulate the release of the incorporated macromolecules, since nanoparticles are vehicles designed for the controlled or delayed release of active substances to human or animal organisms.

Next, for a greater understanding of the characteristics and advantages of the present invention, reference will be made to a series of examples which will explicatively complete the previous description, without in any way meaning that this will be limited thereto.

## EXAMPLES

During the exposition of the following examples, a series of abbreviations will be used:

HANa: Hyaluronic Acid Sodium Salt
CS: Chitosan
TPP: Sodium tripolyphosphate
FITC-BSA: Albumin marked with fluoresceine
CsA: Cyclosporin A
SLF: Simulated lacrimal fluid

### Example 1

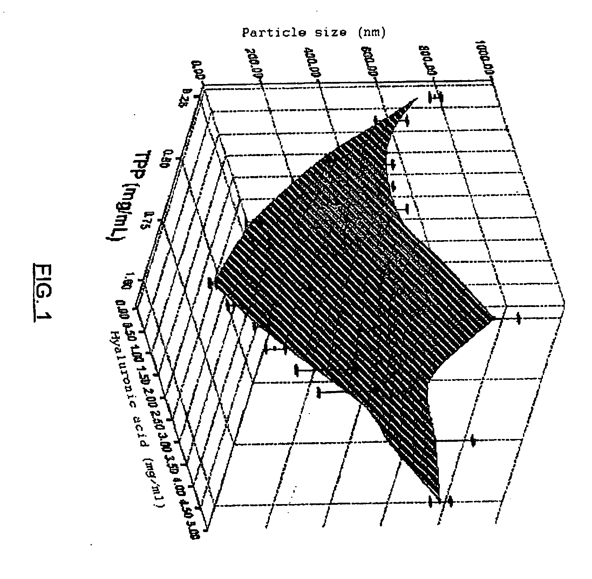

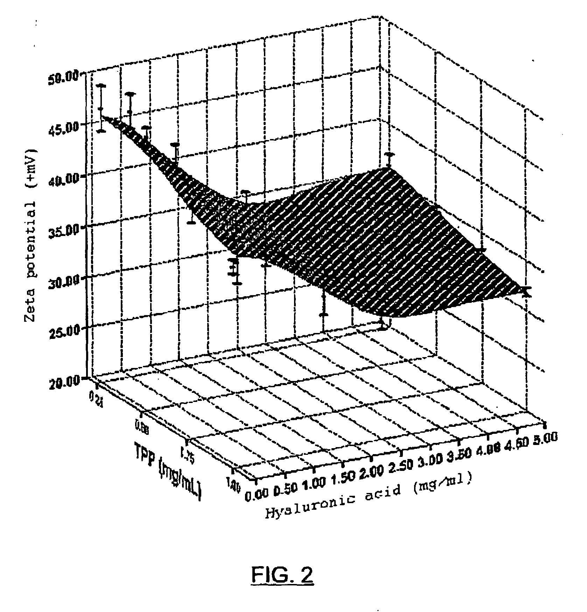

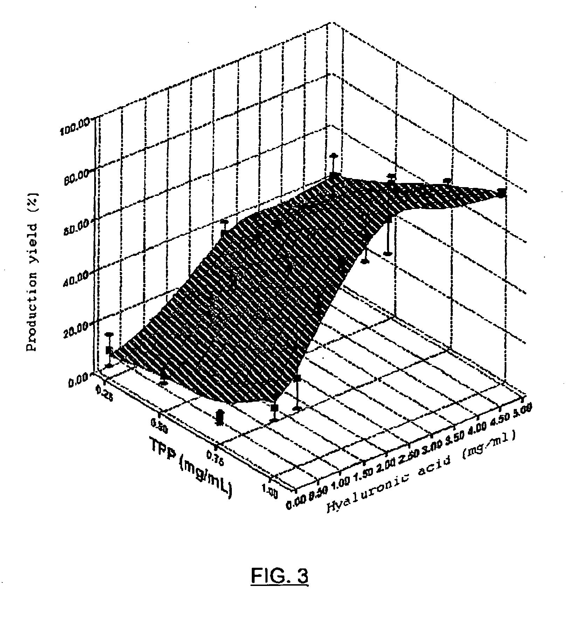

Hyaluronic acid nanoparticles in the form of sodium salt, chitosan as cationic polymer and sodium tripolyphosphate as crosslinking agent, were prepared according to the previously described method. The hyaluronate and sodium tripolyphosphate solution were added to the chitosan solution, with magnetic stirring, which is maintained for half an hour, permitting the complete evolution of the system towards a stable nanoparticulate form. Once prepared, their mean diameter is measured, as well as their surface electric charge (zeta potential) and the production yield is calculated (which is expressed in percentage and takes into account the weight of the nanoparticles with respect to the weight of the incorporated polymers). Table 1 and Figures 1, 2 and 3 show the values which are taken as said parameters in accordance with the proportion of HA-Na, Cs and TPP.

Table 1:

| HA-Na/CS/TPP (w/w) | Mean diameter (nm) | $\zeta$-Potential (1mV) | Production yield |
|---|---|---|---|
| **1/1/0.05** | 769±36 | +36.09±0.99 | 43±0.5 |
| **1/1/0.1** | 696±129 | +34.50±0.28 | 53±3 |
| **1/1/0.15** | 585±9 | +32.90±0.42 | 64±3 |
| **1/1/0.2** | 782±36 | +31.90±0.42 | 75±1 |
| **1/2/0.1** | 550±42 | +34.95±1.14 | 38±4 |

(continued)

| HA-Na/CS/TPP (w/w) | Mean diameter (nm) | ζ-Potential (1mV) | Production yield |
|---|---|---|---|
| 1/2/0.2 | 509±48 | +32.63±0.68 | 55±1 |
| 1/2/0.3 | 584±26 | +32.60±0.52 | 87±8 |
| 1/2/0.4 | 576±100 | +31.66±0.78 | 82±14 |
| 1/3/0.15 | 539±52 | +38.16±0.57 | 19±2 |
| 1/3/0.33 | 442±53 | +32.63±0. 71 | 40±3 |
| 1/3/0.5 | 420±16 | +36.76±0.84 | 58±5 |
| 1/3/0.66 | 379±34 | +35-33±1.93 | 72±3 |
| 1/10/0.5 | 634±55 | 146.11±1.69 | 6±2 |
| 1/10/1 | 396±39 | +44.78±1.55 | 15±1. |
| 7/10/1.5 | 312±29 | +42.0511.42 | 21±6 |
| 1/10/2 | 290±24 | +41.59±2.22 | 34±12 |

## Example 2

Hyaluronic acid nanoparticles in the form of sodium salt, chitosan as cationic polymer and sodium tripolyphosphate as crosslinking agent, were prepared according to the previously described method. A hydrophilic molecule was then incorporated in its composition, selecting FITC-BSA for said purpose. It is a negatively-charged macromolecule in both solutions due to the pH thereof (3 in the case of the chitosan solution and between 8-8.5 in the case of the hyaluronate and tripolyphosphate solutions), for which reason it was incorporated together with the hyaluronic acid to avoid the appearance of interferences in particle formation.

A theoretical charge of 30% was established with respect to the polymer weight, and the encapsulation efficiency was determined (evaluating the free protein by visible spectroscopy, with $\lambda$-494nm) after being prepared according to the method of the invention. Its mean diameter was also measured. The production yield was determined taking into consideration the weight of the polymers and the protein incorporated. Taking into account this last piece of information, it was possible to determine the particles' real charge capacity.

**Table 2**

| HANa/CS/TPP (w/w) | Mean diameter (nm) | Encapsulation efficiency FTTC-BSA (%) | Production yield (%) | Charge in FITC-BSA (%) |
|---|---|---|---|---|
| 1/2/0.4 | 745±58 | 99.75±0.06 | 71±2 | 33 |
| 1/3/0.5 | 518±30 | 99.79±0.03 | 70±3 | 34 |
| 1/10/1.5 | 321±24 | 99.10±0.04 | 36±4 | 63 |

## Example 3

Hyaluronic acid nanoparticles in the form of sodium salt, chitosan as cationic polymer and sodium tripolyphosphate as crosslinking agent, were prepared according to the previously described method. A hydrophobic molecule was then incorporated in its composition, taking for this the polypeptide cyclosporin A, an immunomodulator agent which is practically insoluble in water, especially at moderate temperatures. The preparation method' is the one already disclosed in the present invention, with one modification, since the macromolecule is previously dissolved in a 50% (V/V) acetronitrile/water solution, with a concentration of 10mg/mL. Then, a small volume of this solution, approximately 200 $\mu$L is added to the chitosan solution, and immediately afterwards the solution which contains the hyaluronic acid salt and the crosslinking agent is added. The drug encapsulation has the form of nanocrystals, which justifies the addition process of the second solution being fast, avoiding the macromolecule from precipitating and facilitating the incorporation of nanoparticles.

A theoretical charge of CsA was established at 25% with respect to polymer weight, and once prepared according to the method of the invention, the encapsulation efficiency was determined (evaluating the free polypeptide by ultraviolet spectroscopy, with $\lambda$=200nm). Its mean diameter was also measured. The production yield was determined taking into consideration the weight of the polymers and the polypeptide incorporated. Taking into account this last piece of information, it was possible to determine the particles' real charge capacity.

**Table 3**

| HANa/CS/TPP (w/w) | Mean diameter (nm) | Encapsulation efficiency (%) | Production yield (%) | Charge capacity(%) |
|---|---|---|---|---|
| 1/2/0.4 | 658±43 | 99.68±0.27 | 83±5 | 24 |
| 1/3/0.5 | 536±88 | 99.66±0.25 | 74±6 | 27 |
| 1/10/1.5 | 515±88 | 98.93±0.52 | 54+5 | 37 |

Example 4

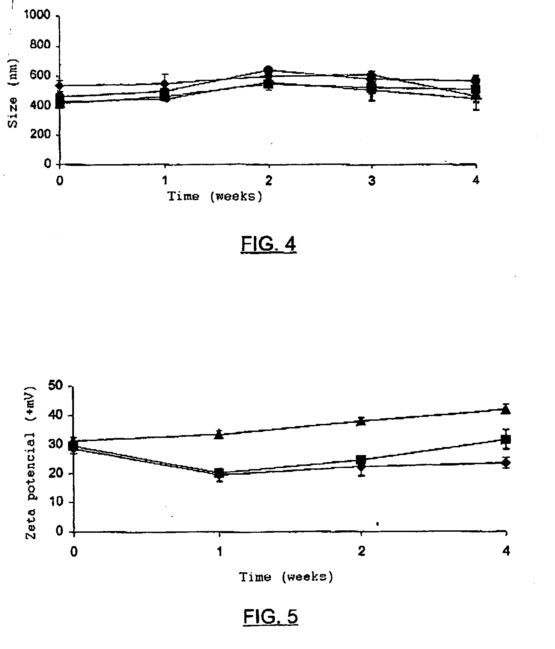

Hyaluronic acid nanoparticles in the form of sodium salt, chitosan as cationic polymer and sodium tripolyphosphate as crosslinking agent, were prepared according to the previously described method. Particle size and surface charge measurements were made, during one month, with the aim of obtaining information on the system evolution with time. For this, different formulations were selected with different quantities of hyaluronic acid. The theoretical HANa/CS/TPP proportions were 1/2/0.4(♦), 1/2.5/0.25(•), 1/3/0.5(■), 1/3/0.66 (-) and 1/10/1.5 (▲). The results presented in figures 4 and 5 showed the little variability of the parameters, size and zeta potential, during the storage.

Example 5

Nanoparticles of hyaluronic acid, chitosan and TPP were prepared according to the present invention. A hydrophobic molecule, CsA, was incorporated in the form described in example 3. Then, the diameter of the nanoparticles was measured throughout one week to check he system stability with time. It has also been verified that the drug is incorporated in the particles and not precipitated in the form of nanocrystals, as no type of crystalline growth was observed. The theoretical charge of CsA was set at a percentage of 25% with respect to the nanoparticle mass. The proportions of the particle-forming polymers and the crosslinking agent, HANa/CS/TPP, were 1/2/0.4 (♦) and 1/3/0.5(■).

Example 6

Hyaluronic acid nanoparticles in the form of sodium salt, chitosan as cationic polymer and sodium tripolyphosphate as crosslinking agent, were prepared according to the previously described method. A proportion of HANa/CS/TPP of 1/2/0.4 was used, and the effect that the type of cryoprotective agent used in the lyophilisation process has on the size was checked on these particles. The influence on the nanoparticle concentration in the suspension to lyophilise was also evaluated. After preliminary assays, two sugars, glucose and trehalose, were selected as cryoprotective agents and their concentration was kept constant, setting it at 5% (w/V).

Example 7

The nanoparticles developed by the method of the present invention, and lyophilised in the presence of 5% (w/V) glucose, were incubated in SLF, which has a pH of 7.4 and a high ion concentration. The formulation selected was the same as in the previous example. Mean diameter measurements were taken of the particles during 24 hours.

Example 8

Hyaluronic acid nanoparticles in the form of sodium salt, chitosan as cationic polymer and sodium tripolyphosphate as crosslinking agent, were prepared according to the previously described method. The formulation developed was that with composition HANa/CS/TPP: 1/2/0.4 and it was lyophilised for 48 hours using 5% glucose as cryoprotective agent. Then, a mucoadhesion study was performed, using SLF and a 4% mucin solution for this.

Hyaluronic acid is a polymer with a viscoelastic behaviour in gel-form. In the case of colloidal suspensions, the reological behaviour is more complex; the viscosity is highly influenced by the particle's surface properties.

The nanoparticles' mucoadhesivity was determined from the following mixtures, prepared aL 50%: nanoparticles/mucin, nanoparticles/SLF and mucin/SLF. The existence of synergism with respect to the first of the mixtures in relation to the sum of the other two, observing the elastic module values (G') and the viscose module (G''), is indicative that the system has mucoadhesive properties. The mathematical formula used was:

$$G'^{('')} = G'^{('')}_{\text{Nanoparticles-4\% mucin}} - ( G'^{('')}_{\text{Nanoparticles-SLF}} + G'^{('')}_{\text{4\% mucin- SLF}})$$

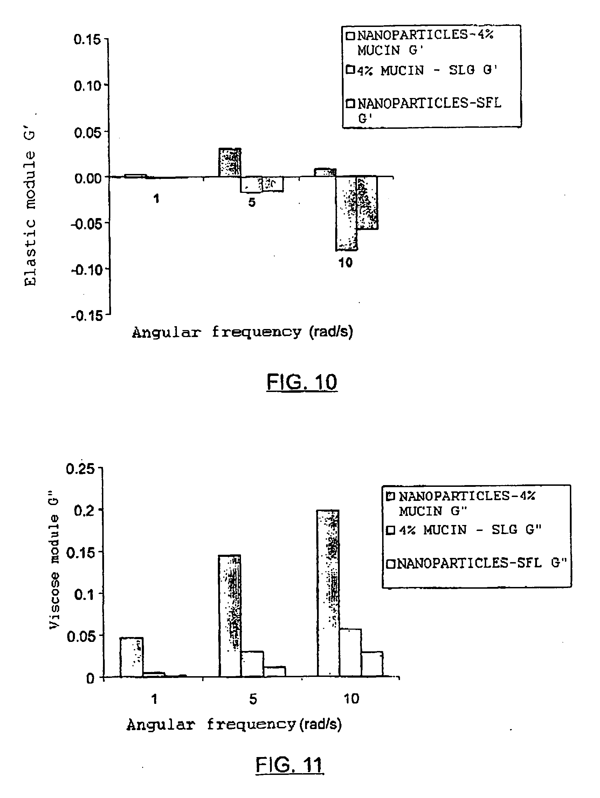

The elastic module and viscose module results appear in figures 10 and 11.

**Claims**

1. Method of obtaining nanoparticles for the administration of at least one active ingredient, with a diameter less than 1μm, **characterised in that** it comprises the steps of:

   a) preparing an aqueous solution of a hyaluronic acid salt;
   b) preparing an aqueous solution of a cationic polymer selected from chitosan, collagen and gelatin;
   c) adding a polyanionic salt to the solution of the hyaluronic acid salt;
   d) stir-mixing the solutions resulting from steps b) and c), spontaneously obtaining the nanoparticles,

   wherein the active ingredient is dissolved in one of resulting solutions a), b) or c) or in the suspension of nanoparticles obtained in step d) to be absorbed in the nanoparticles.

2. Method according to claim 1, **characterised in that** the hyaluronic acid salt solution is prepared at a concentration of between 0.50 and 5 mg/mL.

3. Method according to any of claims 1 and 2, **characterised in that** the cationic polymer solution is prepared at a concentration of between 0.5 and 5 mg/mL.

4. Method according to any of claims 1 to 3, **characterised in that** the anionic salt is added at a concentration of between 0.25 and 1.00 mg/mL.

5. Method according to any of claims 1 to 4, **characterised in that** the active ingredient is a macromolecule.

6. Method according to claim 5, **characterised in that**, if the macromolecule has a lypophilic nature, said macromolecule is dissolved, before incorporating it in one of solutions a) or b), in a mixture of water and a water-miscible organic solvent, so that the concentration of the organic solvent in the end solution is less than 10% by weight.

7. Method according to claim 6, **characterised in that** the organic solvent is acetronitrile.

8. Method according to any of claims 1 to 7, **characterised in that** the hyaluronic acid salt is sodium salt.

9. Method according to any of claims 1 to 8, **characterised in that** the cationic polymer is chitosan.

10. Method according to any of claims 1 to 9, **characterised in that** the cationic polymer is collagen or gelatine.

11. Method according to any of claims 1 to 10, **characterised in that** the polyanionic salt is sodium tripolyphosphate.

12. Method according to any of claims 1 to 11, **characterised in that** the proportion of hyaluronic acid:cationic polymer: polyanionic salt in the end solution is between 1:0.5:0.1 and 1:10:2.

13. Method according to any of claims 1 to 11, **characterised in that** the proportion of hyaluronic acid:cationic polymer: polyanionic salt is between 1:1:0.15 and 1:10:1.5.

14. Method according to any of claims 1 to 13, **characterised in that** it comprises an additional step e), after step d), of lyophilising the nanoparticles obtained in the presence of reduced quantities of sugars.

15. Method according to claim 14, **characterised in that** it comprises an additional step f), after step e), of regenerating the lyophilised nanoparticles.

16. Nanoparticles for the administration of an active ingredient, **characterised in that** it comprises a hyaluronic acid salt, a cationic polymer selected from chitosan, collagen and gelatin, a polyanionic salt and an active ingredient.

17. Nanoparticles according to claim 16 for the administration of an active ingredient, which are obtained by a method as defined in any of claims 1-15.

**18.** Nanoparticles according to claim 17, **characterised in that** the active ingredient is a macromolecule.

**19.** Nanoparticles according to any of claims 17 and 18, **characterised in that** the hyaluronic acid salt is sodium salt.

**20.** Nanoparticles according to any of claims 17 to 19, **characterised in that** the cationic polymer is chitosan.

**21.** Nanoparticles according to any of claims 17 to 19 **characterised in that** the cationic polymer is collagen or gelatine.

**22.** Nanoparticles according to any of claims 17 to 21, **characterised in that** the polyanionic salt is sodium tripolyphosphate.

**23.** Pharmaceutical or cosmetic composition, **characterised in that** it comprises nanoparticles according to claims 16 to 22.

**24.** Pharmaceutical composition according to claim 23 for the topical or parenteral administration on mucous membranes.


**Patentansprüche**

**1.** Verfahren zum Erhalten von Nanopartikeln für die Verabreichung von mindestens einem Wirkstoff mit einem Durchmesser von weniger als 1 $\mu$m, **dadurch gekennzeichnet, dass** es die folgenden Schritte umfasst:

a) Zubereiten einer wässrigen Lösung aus einem Hyaluronsäuresalz;
b) Zubereiten einer wässrigen Lösung aus einem kationischen Polymer ausgewählt aus Chitosan, Collagen und Gelatine;
c) Hinzufügen eines polyanionischen Salzes zu der Lösung aus dem Hyaluronsäuresalz;
d) Verrühren der aus den Schritten b) und C) resultierenden Lösung, spontanes Erhalten der Nanopartikel,

wobei der Wirkstoff in einer der resultierenden Lösungen a), b) oder c) oder in der in Schritt d) erhaltenen Suspension der Nanopartikel gelöst ist, um in die Nanopartikel absorbiert zu werden.

**2.** Verfahren gemäß Anspruch 1, **dadurch gekennzeichnet, dass** die Hyaluronsäuresalz-Lösung in einer Konzentration zwischen 0,50 und 5 mg/mL zubereitet wird.

**3.** Verfahren gemäß irgendeinem der Ansprüche 1 oder 2, **dadurch gekennzeichnet, dass** die kationische Polymerlösung in einer Konzentration zwischen 0,5 und 5 mg/mL zubereitet wird.

**4.** Verfahren gemäß irgendeinem der Ansprüche 1 bis 3, **dadurch gekennzeichnet, dass** das anionische Salz in einer Konzentration zwischen 0,25 und 1,00 mg/mL hinzugefügt wird.

**5.** Verfahren gemäß irgendeinem der Ansprüche 1 bis 4, **dadurch gekennzeichnet, dass** der Wirkstoff ein Makromolekül ist.

**6.** Verfahren gemäß Anspruch 5, **dadurch gekennzeichnet, dass**, falls das Makromolekül lipophiler Natur ist, das Makromolekül in einer Mischung aus Wasser und einem wassermischbaren organischen Lösungsmittel gelöst wird, bevor es in eine der Lösungen a) oder b) eingearbeitet wird, sodass die Konzentration des organischen Lösungsmittels in der Endlösung weniger als 10-Gewichts-% beträgt.

**7.** Verfahren gemäß Anspruch 6, **dadurch gekennzeichnet, dass** das organische Lösungsmittel Acetonitril ist.

**8.** Verfahren gemäß irgendeinem der Ansprüche 1 bis 7, **dadurch gekennzeichnet, dass** das Hyaluronsäuresalz Natriumsalz ist.

**9.** Verfahren gemäß irgendeinem der Ansprüche 1 bis 8, **dadurch gekennzeichnet, dass** das kationische Polymer Chitosan ist.

**10.** Verfahren gemäß irgendeinem der Ansprüche 1 bis 9, **dadurch gekennzeichnet, dass** das kationische Polymer Collagen oder Gellert ist.

**11.** Verfahren gemäß irgendeinem der Ansprüche 1 bis 10, **dadurch gekennzeichnet, dass** das polyanionische Salz Natriumtripolyphosphat ist.

**12.** Verfahren gemäß irgendeinem der Ansprüche 1 bis 11, **dadurch gekennzeichnet, dass** das Verhältnis von Hyaluronsäure : kationischem Polymer : polyanionischem Salz in der Endlösung zwischen 1 : 0,5 : 0,1 und 1 : 10 : 2 ist.

**13.** Verfahren gemäß irgendeinem der Ansprüche 1 bis 11, **dadurch gekennzeichnet, dass** das Verhältnis von Hyaluronsäure : kationischem Polymer : polyanionischem Salz in der Endlösung zwischen 1:1: 0,15 und 1 : 10 : 1,5 ist.

**14.** Verfahren gemäß irgendeinem der Ansprüche 1 bis 13, **dadurch gekennzeichnet, dass** es einen zusätzlichen Schritt e) umfasst, nach Schritt d), bezüglich Lyophilisieren der Nanopartikel, erhalten in Beisein reduzierter Mengen an Zuckern.

**15.** Verfahren gemäß Anspruch 14, **dadurch gekennzeichnet, dass** es einen zusätzlichen Schritt f) umfasst, nach Schritt e), bezüglich Regenerierens der lyophilisierten Nanopartikel.

**16.** Nanopartikel für die Verabreichung eines Wirkstoffs, **dadurch gekennzeichnet, dass** sie ein Hyaluronsäuresalz, ein kationisches Polymer ausgewählt aus Chitosan, Collagen und Gelatine, ein polyanionisches Salz und einen Wirkstoff umfassen.

**17.** Nanopartikel gemäß Anspruch 16 für die Verabreichung eines Wirkstoffs, die Erhalten werden durch das Verfahren, definiert in irgendeinem der Ansprüche 1-15.

**18.** Nanopartikel gemäß Anspruch 17, **dadurch gekennzeichnet, dass** der Wirkstoff ein Makromolekül ist.

**19.** Nanopartikel gemäß irgendeinem der Ansprüche 17 oder 18, **dadurch gekennzeichnet, dass** das Hyaluronsäuresalz Natriumsalz ist.

**20.** Nanopartikel gemäß irgendeinem der Ansprüche 17 bis 19, **dadurch gekennzeichnet, dass** das kationische Polymer Chitosan ist.

**21.** Nanopartikel gemäß irgendeinem der Ansprüche 17 bis 19, **dadurch gekennzeichnet, dass** das kationische Polymer Collagen oder Gelatine ist.

**22.** Nanopartikel gemäß irgendeinem der Ansprüche 17 bis 21, **dadurch gekennzeichnet, dass** das polyanionische Salz Natriumtripolyphosphat ist.

**23.** Pharmazeutische oder kosmetische Zusammensetzung, **dadurch gekennzeichnet, dass** sie Nanopartikel gemäß der Ansprüche 16 bis 22 umfasst.

**24.** Pharmazeutische Zusammensetzung gemäß Anspruch 23, für die topische oder parenterale Verabreichung auf Schleimhäuten.

**Revendications**

**1.** Procédé d'obtention de nanoparticules pour l'administration d'au moins un principe actif, avec un diamètre inférieur à 1$\mu$m, **caractérisé en ce qu'**il comprend les étapes consistant à :

a) préparer une solution aqueuse d'un sel d'acide hyaluronique ;
b) préparer une solution aqueuse d'un polymère cationique sélectionné parmi le chitosane, le collagène et la gélatine ;
c) ajouter un sel polyanionique à la solution du sel d'acide hyaluronique ;
d) mélanger en agitant les solutions résultant des étapes b) et c), obtenant spontanément les nanoparticules,

dans lequel le principe actif est dissous dans l'une des solutions résultantes a), b) ou c) ou dans la suspension de nanoparticules obtenues à l'étape d) pour être absorbé dans les nanoparticules.

2. Procédé selon la revendication 1, **caractérisé en ce que** la solution de sel d'acide hyaluronique est préparée à une concentration entre 0,50 et 5 mg/ml.

3. Procédé selon l'une quelconque des revendications 1 et 2, **caractérisé en ce que** la solution de polymère cationique est préparée à une concentration entre 0,5 et 5 mg/ml.

4. Procédé selon l'une quelconque des revendications 1 à 3, **caractérisé en ce que** le sel anionique est ajouté à une concentration entre 0,25 et 1,00 mg/ml.

5. Procédé selon l'une quelconque des revendications 1 à 4, **caractérisé en ce que** le principe actif est une macromolécule.

6. Procédé selon la revendication 5, **caractérisé en ce que**, si la macromolécule a une nature lipophile, ladite macromolécule est dissoute, avant son incorporation dans l'une des solutions a) ou b), dans un mélange d'eau et de solvant organique miscible dans l'eau, de sorte que la concentration du solvant organique dans la solution finale est inférieure à 10 % en poids.

7. Procédé selon la revendication 6, **caractérisé en ce que** le solvant organique est l'acétonitrile.

8. Procédé selon l'une quelconque des revendications 1 à 7, **caractérisé en ce que** le sel d'acide hyaluronique est le sel de sodium.

9. Procédé selon l'une quelconque des revendications 1 à 8, **caractérisé en ce que** le polymère cationique est le chitosane.

10. Procédé selon l'une quelconque des revendications 1 à 9, **caractérisé en ce que** le polymère cationique est le collagène ou la gélatine.

11. Procédé selon l'une quelconque des revendications 1 à 10, **caractérisé en ce que** le sel polyanionique est le tripolyphosphate de sodium.

12. Procédé selon l'une quelconque des revendications 1 à 11, **caractérisé en ce que** la proportion d'acide hyaluronique/polymère cationique/sel polyanionique dans la solution finale se situe entre 1/0,5/0,1 et 1/10/2.

13. Procédé selon l'une quelconque des revendications 1 à 11, **caractérisé en ce que** la proportion d'acide hyaluronique/polymère cationique/sel polyanionique se situe entre 1/1/0,15 et 1/10/1,5.

14. Procédé selon l'une quelconque des revendications 1 à 13, **caractérisé en ce qu'**il comprend une étape supplémentaire e), après l'étape d), de lyophilisation des nanoparticules obtenues en présence de quantités réduites de sucres.

15. Procédé selon la revendication 14, **caractérisé en ce qu'**il comprend une étape supplémentaire f), après l'étape e), de régénération des nanoparticules lyophilisées.

16. Nanoparticules pour l'administration d'un principe actif, **caractérisées en ce qu'**elles comprennent un sel d'acide hyaluronique, un polymère cationique sélectionné parmi le chitosane, le collagène et la gélatine, un sel polyanionique et un principe actif.

17. Nanoparticules selon la revendication 16 pour l'administration d'un principe actif, qui sont obtenues par un procédé tel que défini dans l'une quelconque des revendications 1 à 15.

18. Nanoparticules selon la revendication 17, **caractérisées en ce que** le principe actif est une macromolécule.

19. Nanoparticules selon l'une quelconque des revendications 17 et 18, **caractérisées en ce que** le sel d'acide hyaluronique est le sel de sodium.

**20.** Nanoparticules selon l'une quelconque des revendications 17 à 19, **caractérisées en ce que** le polymère cationique est le chitosane.

**21.** Nanoparticules selon l'une quelconque des revendications 17 à 19, **caractérisées en ce que** le polymère cationique est le collagène ou la gélatine.

**22.** Nanoparticules selon l'une quelconque des revendications 17 à 21, **caractérisées en ce que** le sel polyanionique est le tripolyphosphate de sodium.

**23.** Composition pharmaceutique ou cosmétique, **caractérisée en ce qu'**elle comprend des nanoparticules selon les revendications 16 à 22.

**24.** Composition pharmaceutique selon la revendication 23 pour l'administration topique ou parentérale sur des membranes muqueuses.

FIG. 1

**FIG. 2**

FIG. 3

FIG. 4

FIG. 5

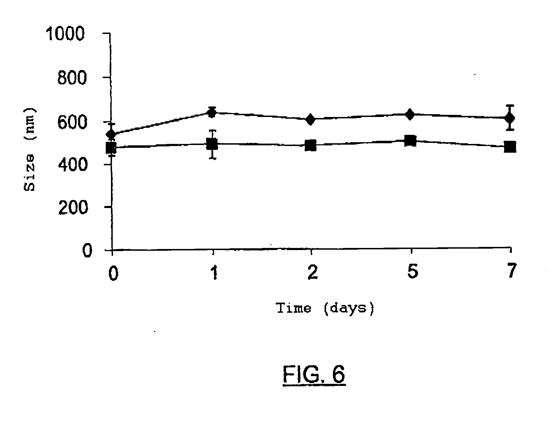

FIG. 6

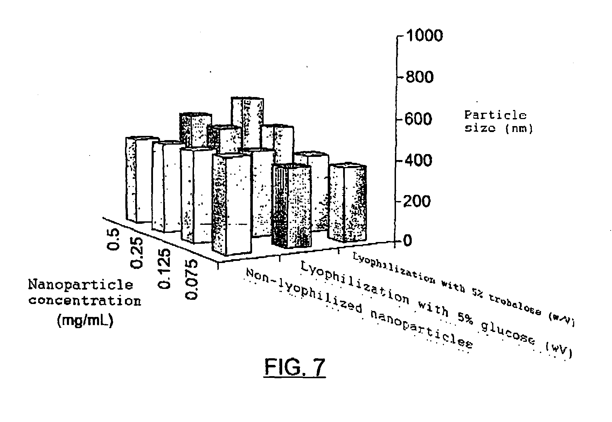

FIG. 7

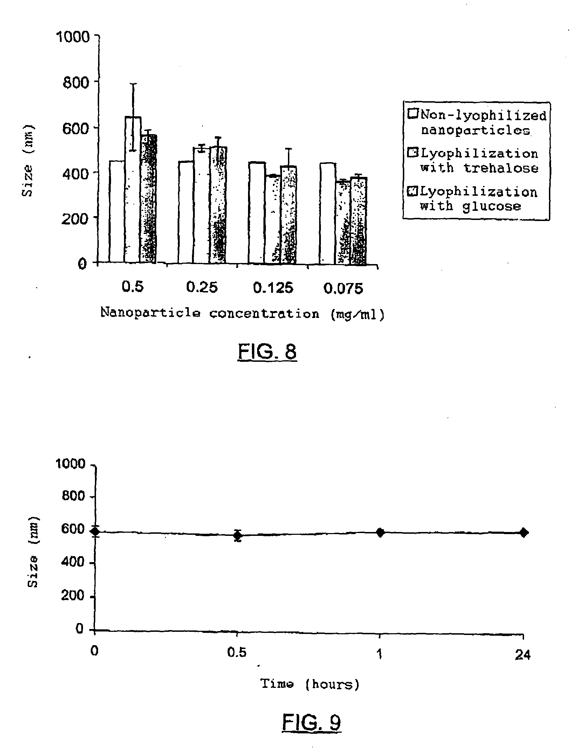

FIG. 8

FIG. 9

FIG. 10

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9606622 A
- US 6383478 B
- WO 0101964 A
- WO 904747 A
- EP 0544259 A
- WO 018274 A
- US 4582865 A
- WO 0128602 A
- WO 9009401 A

- WO 0230990 A
- WO 8903207 A
- US 6066340 A
- US 2001053359 A
- US 2002197328 A
- US 20030026844 A
- WO 9947130 A
- US 6132750 A
- WO 9918934 A

**Non-patent literature cited in the description**

- **Sand et al.** *Acta Ophthalmol.,* 1989, vol. 67, 181-183
- **Nishida et al.** *Exp. Eye Res,* 1991, vol. 53, 753-758

- **Benedetti et al.** *Journal of Controlled Release,* 1990, vol. 13, 33-41
- **Lim et al.** *J. Controll. Rel.,* 2000, vol. 66, 281-292
- **Lim et al.** *Tnt. J. Pharm.,* 2002, vol. 23, 73-82